(19) Europäisches Patentamt
European Patent Office
Office européen des brevets

(11) **EP 2 450 440 A1**

(12) **EUROPEAN PATENT APPLICATION**
published in accordance with Art. 153(4) EPC

(43) Date of publication:
**09.05.2012 Bulletin 2012/19**

(51) Int Cl.:
*C12N 9/64* (2006.01)    *C12N 15/57* (2006.01)
*A61K 38/48* (2006.01)    *A61P 7/02* (2006.01)

(21) Application number: **10793558.7**

(22) Date of filing: **14.06.2010**

(86) International application number:
**PCT/CN2010/073987**

(87) International publication number:
**WO 2011/000263 (06.01.2011 Gazette 2011/01)**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **02.07.2009   CN 200910094685**

(71) Applicant: **Kunming Institute of Zoology Chinese
Academy of
Sciences
Yunnan 650223 (CN)**

(72) Inventors:
• **LAI, Ren
  Kunming
  Yunnan 650223 (CN)**
• **MA, Dongying
  Kunming
  Yunnan 650223 (CN)**

(74) Representative: **Kramer - Barske - Schmidtchen
European Patent Attorneys
Landsberger Straße 300
80687 München (DE)**

(54) **FIBRINOGENOLYTIC ENZYME TABFIBLYSIN OF HORSEFLY, TABANUS  YAO, ENCODING GENE AND USE THEREOF**

(57)    A horsefly protease tabfiblysin isolated from the salivary gland of the horsefly, Tabanus Yao, a gene encoding the protease and use thereof are disclosed by the present invention. It belongs to the technical field of biomedicine. The molecular weight of the horsefly protease tabfiblysin is 27145.5 Daltons. Its full-length sequence is composed of 255 amino acids. Its encoding sequence is composed of 768 nucleotides. The horsefly protease tabfiblysin can hydrolyze fibrinogen and inhibit the aggregation of blood platelet dramatically, and can be used for preparing the drug for treating thrombotic diseases.

Fig.3

SEQUENCE LISTING

<110> KUNMING INSTITUTE OF ZOOLOGY CHINESE ACADEMY OF SCIENCES
<120> FIBRINOGENOLYTIC ENZYME TABFIBLYSIN OF HORSEFLY, TABANUS YAO, ENCODING
      GENE AND USE THEREOF
<130> 20100610

<150> CN 200910094685.X
<151> 2009-07-02

<160> 2
<170> PatentIn version 3.3

<210> 1
<211> 768
<212> DNA
<213> Tabacus yao Macquart

<220>
<221> CDS
<222> (1)..(765)

<400> 1
```
atg act tcc ata ccg gtg tcc agc ttt tta ctt gcg gcc tta gta cta       48
Met Thr Ser Ile Pro Val Ser Ser Phe Leu Leu Ala Ala Leu Val Leu
1               5                   10                  15

caa tac gcc act agc gac gca gtt aac tac tgt aga ttg cct tgt cga       96
Gln Tyr Ala Thr Ser Asp Ala Val Asn Tyr Cys Arg Leu Pro Cys Arg
            20                  25                  30

ggg gat aat tac cat gtg ggc tgt ggt gag cca gcg tat gcc cag gaa      144
Gly Asp Asn Tyr His Val Gly Cys Gly Glu Pro Ala Tyr Ala Gln Glu
        35                  40                  45

tgt ggg caa agt cca aga act cgc gag tta ttg aag gag cat aga aat      192
Cys Gly Gln Ser Pro Arg Thr Arg Glu Leu Leu Lys Glu His Arg Asn
    50                  55                  60

gag att ttg tcg aag atc aac gat gtg cga gac cat gtg gcc aag gga      240
Glu Ile Leu Ser Lys Ile Asn Asp Val Arg Asp His Val Ala Lys Gly
65                  70                  75                  80

tca tgg gga cta cca gtg gca gcc cga atg aaa gtt gtt gta tgg gat      288
Ser Trp Gly Leu Pro Val Ala Ala Arg Met Lys Val Val Val Trp Asp
                85                  90                  95

gcg gaa ctc gct ggt tta gcc aaa cga cat aca aag gga tgc gtt gga      336
Ala Glu Leu Ala Gly Leu Ala Lys Arg His Thr Lys Gly Cys Val Gly
            100                 105                 110

gag aca cat gcg tgt cga aac aca gaa cgt ttc tgg ttg cca ggt caa      384
Glu Thr His Ala Cys Arg Asn Thr Glu Arg Phe Trp Leu Pro Gly Gln
        115                 120                 125

cta aac ttc aaa tac tcc ggc gac aag cta ccc cgc att aag gaa ctt      432
Leu Asn Phe Lys Tyr Ser Gly Asp Lys Leu Pro Arg Ile Lys Glu Leu
    130                 135                 140

att gat gat gct gtc aaa aaa ggc cac ttg cag aag cat aat att acg      480
Ile Asp Asp Ala Val Lys Lys Gly His Leu Gln Lys His Asn Ile Thr
145                 150                 155                 160

aga gaa att ata ggg aac tat agg gag aat gga cct aat ggg gat gtc      528
```

```
Arg Glu Ile Ile Gly Asn Tyr Arg Glu Asn Gly Pro Asn Gly Asp Val
              165                 170                 175

aag gaa ctt gcc ttg gcc ata tct gat cga gtc acc gcc gtt ggc tgc        576
Lys Glu Leu Ala Leu Ala Ile Ser Asp Arg Val Thr Ala Val Gly Cys
              180                 185                 190

gga cta act act tgg cag gat gga gca aag gct cgc gca ctt ctt acg        624
Gly Leu Thr Thr Trp Gln Asp Gly Ala Lys Ala Arg Ala Leu Leu Thr
              195                 200                 205

tgt aac ttc tcc tcg cag aac act cgg ggt cga cct gtc tac aaa atc        672
Cys Asn Phe Ser Ser Gln Asn Thr Arg Gly Arg Pro Val Tyr Lys Ile
          210                 215                 220

ggc aat agt cct ggc gaa aag tgc atc gag aag gat gaa acg tat aaa        720
Gly Asn Ser Pro Gly Glu Lys Cys Ile Glu Lys Asp Glu Thr Tyr Lys
      225                 230                 235                 240

aac ttg tgc cct gct act gaa cct atc gac cct aat aag tct aac tag        768
Asn Leu Cys Pro Ala Thr Glu Pro Ile Asp Pro Asn Lys Ser Asn
                  245                 250                 255
```

```
<210>   2
<211>   255
<212>   PRT
<213>   Tabacus yao Macquart


<400>   2

Met Thr Ser Ile Pro Val Ser Ser Phe Leu Leu Ala Ala Leu Val Leu
1                   5                   10                  15


Gln Tyr Ala Thr Ser Asp Ala Val Asn Tyr Cys Arg Leu Pro Cys Arg
              20                  25                  30


Gly Asp Asn Tyr His Val Gly Cys Gly Glu Pro Ala Tyr Ala Gln Glu
          35                  40                  45


Cys Gly Gln Ser Pro Arg Thr Arg Glu Leu Leu Lys Glu His Arg Asn
      50                  55                  60


Glu Ile Leu Ser Lys Ile Asn Asp Val Arg Asp His Val Ala Lys Gly
65                  70                  75                  80


Ser Trp Gly Leu Pro Val Ala Ala Arg Met Lys Val Val Val Trp Asp
                  85                  90                  95


Ala Glu Leu Ala Gly Leu Ala Lys Arg His Thr Lys Gly Cys Val Gly
              100                 105                 110


Glu Thr His Ala Cys Arg Asn Thr Glu Arg Phe Trp Leu Pro Gly Gln
          115                 120                 125
```

```
Leu Asn Phe Lys Tyr Ser Gly Asp Lys Leu Pro Arg Ile Lys Glu Leu
    130             135             140

Ile Asp Asp Ala Val Lys Lys Gly His Leu Gln Lys His Asn Ile Thr
145             150             155             160

Arg Glu Ile Ile Gly Asn Tyr Arg Glu Asn Gly Pro Asn Gly Asp Val
                165             170             175

Lys Glu Leu Ala Leu Ala Ile Ser Asp Arg Val Thr Ala Val Gly Cys
            180             185             190

Gly Leu Thr Thr Trp Gln Asp Gly Ala Lys Ala Arg Ala Leu Leu Thr
        195             200             205

Cys Asn Phe Ser Ser Gln Asn Thr Arg Gly Arg Pro Val Tyr Lys Ile
    210             215             220

Gly Asn Ser Pro Gly Glu Lys Cys Ile Glu Lys Asp Glu Thr Tyr Lys
225             230             235             240

Asn Leu Cys Pro Ala Thr Glu Pro Ile Asp Pro Asn Lys Ser Asn
            245             250             255
```

**Description**

**Technical field**

This invention relates to a fibrinogenolytic enzyme tabfiblysin of horsefly, tabanus Yao, and its encoding gene and application. It belongs to the technical field of biomedicine.

**Background of the invention**

Thrombotic diseases are a type of disease that seriously affects human health. In particular, cardiac and brain blood vessel thrombotic diseases have become the first cause of death in China. Timely use of anti-thrombus drugs can greatly increase patient persistence and reduce extent and scope of harm to viscera. Such anti-thrombus drugs mainly include those that dissolve thrombus, anticoagulant drugs, and anti-platelet drugs. Existing anticoagulant drugs and anti-platelet drugs have side effects of hemorrhage, thrombopenia, and cease of effect after stop of use of the drug. Therefore, people are actively looking for various new highly efficient, distinctive, and low toxicity anti-thrombus drugs from biological sources.

Gadfly family of insect is commonly referred to as horsefly. This insect belongs to arthropoda phylum diptera order brachycera suborder gadfly family. It is an important insect in medicine and theriatrics. Its adult is a traditional Chinese medicinal material commonly referred to as gadfly and features promotion of blood circulation to dispel blood stasis and breaking of dysmenorrheal. Use of this drug has a long history and it can provide drone and revelation for development of new modern drug. On the other hand, complexity of compositions of traditional Chinese medicine and method of processing of such medicine limit functioning of its active constituents. For this reason, locating specific active monomer in such traditional medicines is an important content of modernization of traditional Chinese medicines.

**Summary of the invention**

This invention aims at provision of fibrinogenolytic enzyme of horsefly, tabanus Yao, and its encoding gene and application, based on existing technologies. Technical scheme of this invention is described below.

Tabanus Yao protease tabfiblysin is an active protein separated by us from salivary gland of tabanus Yao for the first time in the world. The molecular weight of this protease is 27145.5 Daltons. Its full-length sequence primary structure is the amino acid sequence shown by SEQ ID NO: 2.

Cloning of genes of tabanus Yao salivary gland protease tabfiblysin includes: extraction of total RNA of tabanus Yao salivary gland, purification of mRNA, mRNA reverse transcription, and construction of cDNA library. Primer is designed and PCR method is used to screen encoding gene. Results of gene sequencing show that tabfiblysin encoding sequence is composed of 768 nucleotides. Sequence from end 5' to end 3' is the nucleotide sequence shown by SEQ ID NO:1.

Tabanus Yao salivary gland protease tabfiblysin of this invention has apparent effect of hydrolysis of fibrinogen and suppression of platelet aggregation, and can be used as a drug to treat thrombotic diseases.

**Description of drawing figures**

**[0008]**

　　Fig. 1 shows Sephadex G-75 filtration chromatography of tabanus Yao salivary gland homogenate (SGE).

In this figure, the arrow points to active peak.

　　Fig. 2 shows SDS-PAGE analysis of fibrinogen hydrolysis effect of this invention.

　　Fig. 3 shows effect of tabfiblysin on human platelet aggregation induced by ADP.

**Preferred embodiments**

**Preferred embodiment I: Cloning of gene of tabanus Yao salivary gland protease tabfiblysin**

I. Extraction of total RNA of tabanus Yao salivary gland:

A. Perform vivisection of tabanus Yao (from Shanxi Province of China; scientific name is *T*. yao Macquart). Pick out the salivary gland and instantly place it in liquid nitrogen. Quickly take certain amount (0.5-2 mg) of salivary gland tissue and add it into precooled Trizol extraction buffer solution (product of Invitrogen, USA). Place the mixture on ice for homogenate for 15 min.

B. Add chloroform of 1/5 the volume of Trizol and shake fiercely for 15 s. Place the mixture under room temperature for 5 min. Perform centrifugation at 4 °C and 12000 rpm for 10 min. Take supernatant.

C. Add the supernatant in iso-propyl alcohol of the same volume, and place the mixture under room temperature for 10 min. Perform centrifugation at 4 °C and 12000 rpm for 10 min. After precipitation, use 75 % alcohol to wash once. Allow airing; precipitate at tube bottom is total RNA of tabanus Yao salivary gland.

II. Purification of tabanus Yao salivary gland mRNA:

Separation and purification of tabanus Yao salivary gland mRNA adopts PolyATtract® mRNA Isolation Systems kit of PROMEGA, USA.

A. Take 500 $\mu$g of tabanus Yao salivary gland total RNA and dissolve it in 500 $\mu$L DEPC. Place the solution in 65 °C water bath for 10 min. Then, add 3 $\mu$L of Oligo (dT) probe and 13 $\mu$L of 20×SSC solution. Mix even. Place the mixture under room temperature for cooling. The resultant solution is referred to as liquid A.

B. Washing of magnetic beads (SA-PMP): Lightly tap magnetic beads to mix them even. Move them to the magnetic rack for 30 s of adsorption. Discard supernatant. Add 300 $\mu$L of 0.5×SSC. Move the lot to the magnetic rack for 30 s of adsorption. Finally, add 100 $\mu$L of 0.5×SSC. The resultant liquid is referred to as liquid B.

C. Add liquid A into liquid B and place the mixture under room temperature for 10 min. Move this mixture to the magnetic rack for 30 s of adsorption. Discard supernatant. Wash 4 times using 0.1×SSC. Finally, discard supernatant. Add 100 $\mu$L of DEPC water suspension. Move the lot onto the magnetic rack for 30 s of adsorption. Transfer the supernatant to a new test tube and add 150 $\mu$L of DEPC water suspension. Move this to magnetic rack for 30 s of adsorption. Transfer supernatant to aforesaid test tube; this is purified tabanus Yao salivary gland mRNA.

D. Add 3 M sodium acetate (pH 5.2) of 1/10 volume and equal volume of iso-propyl alcohol. Place the lot at -70 °C for 30 min. Perform centrifugation at 4 °C and 12000 rpm for 10 min. Discard supernatant. Dissolve the precipitate in 10 $\mu$L of DEPC.

III. Construction of tabanus Yao salivary gland cDNA library: Creator™ SMART ™ cDNA Library Construction Kit plasmid cDNA library construction kit of CLONTECH is adopted.

A. Synthesis of cDNA first chain (reverse transcription of mRNA):

1. In sterile PCR tube, add 1 $\mu$L of tabanus Yao salivary gland mRNA, 1 $\mu$L of SMART IV primer, 1 $\mu$L of CDS III/3'PCR primer, and 2 $\mu$L of deionized water, so that total volume reaches 5 $\mu$L.

2. Mix the lot even and perform short centrifugation. Place it at 72 °C for 2 min.

3. Place the centrifuge tube on ice for 2 min of incubation.

4. In the centrifuge tube, add the following reagents: 2.0 $\mu$L of 5× first chain buffer, 1.0 $\mu$L of 20 mM DTT, 1.0 $\mu$L of 10 mM dNTP mixture, and 1.0 $\mu$L of PowerScript reverse transcriptase.

5. Mix reagents in the centrifuge tube and perform short centrifugation. Place it at 42 °C for 1 hour.

6. Place the centrifuge tube on ice to interrupt synthesis of the first chain.

7. Take 2 $\mu$L of synthesized cDNA first chain from the centrifuge tube for later use.

B. Amplify to obtain second chain using the method of long terminal polymerase chain reaction (LD- PCR)

1. Preheat PCR instrument at 95 °C.

2. Place 2 μL of cDNA first chain (mRNA reverse transcription), 80 μL of deionized water, 10 μL of 10×Advantage 2 PCR buffer, 2 μL of 50×dNTP mixture, 2 μL of 5'PCR primer, 2 μL of CDS III/3' PCR primer, and 2 μL of bacillus coli polymerase in centrifuge tube for reaction.

3. In PCR instrument, perform amplification in the following sequence: 20 s at 95 °C; 22 cycles: 5 s at 95 °C followed by 6 min at 68 °C.

4. After the cycles, extract synthesized cDNA dual chain in the centrifuge tube.

C. Preparation of bacillus coli DH5α competent cells:

1. Select single DH5α colony (purchased from Beijing Tiangeng Biochemical Science & Technology Co. Ltd.) and inoculate it to 3 mL of LB culture medium that does not contain ampicillin. Culture overnight at 37 °C. In the next day, take aforesaid bacterium liquid and inoculate it to 50 mL of LB culture medium in proportion of 1: 100. Oscillate for 2 hours at 37 °C. When $OD_{600}$ value reaches 0.35, harvest bacterial culture. Place it in ice bath for 10 min. Centrifuge it at 4 °C and 5000 rpm for 10 min, to recover cells.

2. Add 600 μL of pre-cooled 0.1 mol/L $CaCl_2$-$MgCl_2$ solution heavy suspension in each mL of initial culture liquid.

3. Carry out centrifugation at 4 °C and 5000 rpm for 10 min to recover cells.

4. Add 60 μL of pre-cooled 0.1 mol/L $CaCl_2$ heavy suspension cell precipitate in each mL of initial culture, and sub-pack resultant liquid for later use.

D. Enzyme cutting, connection, and transformation of connection product:

1. In micro-centrifugal tube, add 1 μL of pMD19-T carrier (purchased from Takara, Japan) and 4 μL of tabanus Yao salivary gland cDNA dual chain solution. Full dose is 5 μL.

2. Add 5 μL (tales dose) of ligase buffer mixture.

3. Allow reaction for 2 hours at 16 °C.

4. Add full dose (10 μL) to 100 μL of DHSa competent cells and place the lot in ice bath for 30 min.

5. After heating at 42 °C for 90 s, place it again in ice for 1 min.

6. Add 900 μL of LB culture medium that had been bathed at 37 °C; slowly shake it at 37 °C for 60 min of cultivation.

7. Take 200 μL of the above and apply it on LB culture medium containing X-Gal, IPTG, and Amp at 37 °C for 16 hours of cultivation, to form single colony.

8. In each LB flat dish, use 5 mL of LB liquid culture medium to wash colony. Add 30 % glycerin for frozen storage. cDNA constructed contains about $1×10^6$ separate clones.

IV. Tabanus Yao salivary gland protease tabfiblysin gene order measurement and results:

According to inner peptide amino acid sequence of tabanus Yao salivary gland protease tabfiblysin obtained by separation and purification, and preference of dipster codon, we have designed a degenerate primer NT3-F21, to match joint primer CDSIII used for construction of tabanus Yao salivary gland cDNA library. Positive primer sequence and reverse primer sequence are:

NT3-F21: 5'-Tac tcc gg(g/c) gac aa(a/g) ct(g/a) cc(c/g) cgc-3';
CDSIII primer: 5'-att cta gag gcc gag gcg gcc atg-3'

In which two basic groups in parentheses indicate degenerate primer.

Take NT3-F21 and 3'PCR as primer, in constructed tabanus Yao salivary gland cDNA library, screen clone of cDNA sequence in which code tabfiblysin is inserted.

Reverse primer is designed according to measured nucleotide sequence of mature protein, and 5' end conservative sequence cloned by cDNA library constructed is used as positive primer. Positive primer sequence and reverse primer sequence are:

> 5'primer: 5'-aag cag tgg tat caa cgc aga gt-3'

> NT3-R21: 5'-(c/g)gg ( t/c)ag (c/t)tt gtc (g/c)cc gga gta-3'

Perform PCR with tabanus Yao salivary gland cDNA constructed as template. Reaction conditions: pre-denaturation at 95 °C for 4 min, followed by 35 cycles at the following conditions: 30 s at 94 °C, 30 s at 58 °C, and 40 s at 72 °C. Next, place at 72 °C for 10 min. Using this method, in tabanus Yao salivary gland cDNA library constructed, screen cDNA sequence in which code Tabfiblysin is inserted. Measurement results show that cDNA sequence of code tabfiblysin is composed of 768 nucleotides. Sequence from 5' end to 3' end is the nucleotide sequence shown by SEQ ID NO:1.

**Preferred embodiment II: Preparation of tabanus Yao salivary gland protease tabfiblysin**

I. Tabanus Yao salivary gland dissection, homogenate, and treatment

Method of dissection of tabanus Yao (from Shanxi Province of China; scientific name is *T*. yao Macquart): Use small scissors to cut open back of the insect along its centerline. Soak it in 4 °C 0.01 M phosphate buffer (pH 7.2) containing 0.15 M NaCl. Pick out the salivary gland and place it in a beaker containing aforesaid buffer solution (at -20 °C). After dissection, carry out centrifugation at 10000 rpm for 10 min. Continue to store the supernatant at -20 °C. After thorough homogenate in precipitation glass homogenizer on ice, carry out centrifugation at 10000 rpm for 10 min. Combine supernatants from these two centrifugation operations, carry out freeze drying, and take a sample for dissolution in water, desalination by Sephadex G-25, and freeze drying.

II. For each step of separation and purification, activity is followed up using fibrinogen hydrolysis method.

> Step 1: Sephadex G-75 gel filtration: Dissolve tabanus Yao salivary gland homogenate freeze-dried powder in 0.05 M Tris-HCl + 0.1 M NaCl buffer solution (pH 7.2). Apply a sample of this solution to Sephadex -75 (product of Pharmacia) solvent resistant column (1000 mm long; dia. of 26 mm). Use the same solution for elution and collect active constituents.

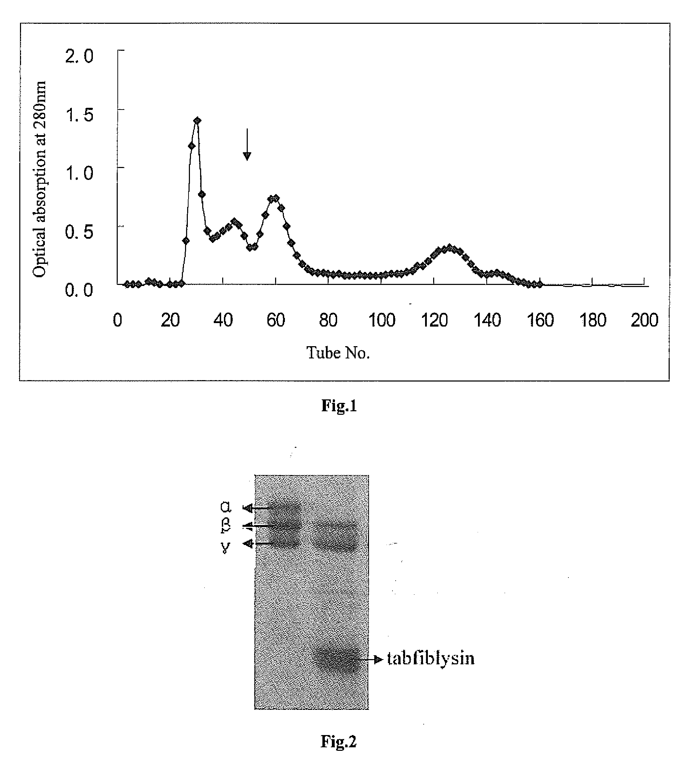

> Step 2: FPLC Resource S ion exchange chromatography: Freeze dry active constituent peak (Fig. 1) obtained by Sephadex G-75 gel filtration and dissolve it in water. Carry out dialysis of the same in 0.05 M Tris-HC1 pH 8.3 buffer solution for 12 hr. Apply a sample thereof to Resource S column, and use 0-0.5 M NaCl linear gradient for elution. Purification yields fibrinogen hydrolytic enzyme. This step is performed in AKTA Purifier FPLC system.

**Preferred embodiment III: Pharmacological experiments of tabanus Yao salivary gland protease**

I. Detection of fibrinogen hydrolytic activity

Take 10 $\mu$L of 2 mg/mL fibrinogen (purchased from Sigma of USA and dissolved in 50 mM Tris-HCl, pH 7.4 buffer solution containing 150 mM NaCl), place it at 37 °C for 24 hr along with the sample. Carry out 12 % SDS-PAGE reducing electrophoresis analysis, as shown in Fig. 2. Results show that this enzyme can apparently hydrolyze $\alpha$-chain of fibrinogen, but its hydrolysis activity for $\beta$-chain is low and it cannot hydrolyze $\gamma$-chain. Its hydrolysis activity sequence is $\alpha > \beta > \gamma$.

II. Platelet aggregation suppression experiment

Platelet of rich plasma (PRP) aggregation suppression experiment: Dilute healthy person platelet by plasma to $2.5 \times 10^8$ pieces/mL. Take 300 $\mu$L of PRP and add suitable amount of sample; keep the mixture warm for 5 min and then add ADP to induce aggregation. Record aggregation of platelet after 5 min.

Calculation of aggregation suppression rate I:

$$I = B/A \times 100\%$$

A = maximum aggregation that can be reached by excitant (in transmittance, %);

B = maximum aggregation that can be reached by excitant after use of the sample

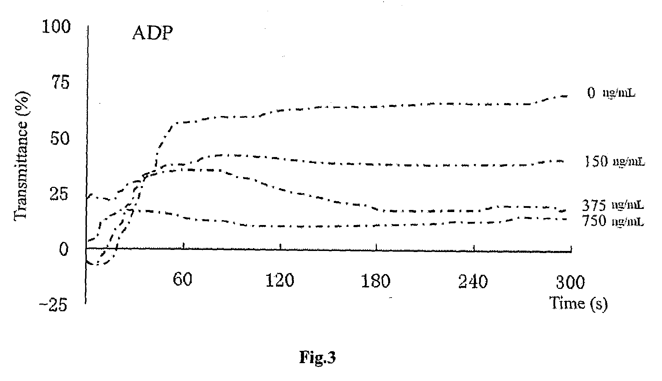

Results show that tabfiblysin can apparently suppress platelet aggregation induced by ADP and the suppression rate increases with increase of inhibitor concentration (Fig.3).

III. Detection of hemorrhage activity

Dissolve the sample in normal saline so that the concentration is 0.6 mg/mL. Take 30 $\mu$L of this solution for hypodermic injection of ICR mice (of body weight of 18-22 g; provided by Kunming Medical College Experimental Animal Center). After 24 hours, peel off mouse skin and observe size of endothelium blood spots. Compare results with a group of mice that adopt normal saline. Results show that tabfiblysin dose as high as 18 $\mu$g/mouse will not cause subcutaneous hemorrhage.

Pharmacological experiments of tabanus Yao salivary gland protease show that tabanus Yao salivary gland protease tabfiblysin features apparent effect of hydrolysis of fibrinogen and suppression of platelet aggregation. Tabfiblysin can be used to treat thrombotic diseases.

## Claims

1. Tabanus Yao protease tabfiblysin, an active protein separated from tabanus Yao salivary gland, of molecular weight of 27145.5 Daltons, and full-length sequence primary structure of the amino acid sequence shown by SEQ ID NO:2.

2. Gene of tabanus Yao protease tabfiblysin, composed of 768 nucleotides, with its 5' end to 3' end sequence being the nucleotide sequence shown by SEQ ID NO:1.

3. Use of tabanus Yao protease tabfiblysin, which can hydrolyze fibrinogen and suppress platelet aggregation, without hemorrhage activity, to treat thrombotic diseases.

Fig.1

Fig.2

Fig.3

# INTERNATIONAL SEARCH REPORT

| | International application No. |
| --- | --- |
| | PCT/CN2010/073987 |

**A. CLASSIFICATION OF SUBJECT MATTER**

See extra sheet

According to International Patent Classification (IPC) or to both national classification and IPC

**B. FIELDS SEARCHED**

Minimum documentation searched (classification system followed by classification symbols)

IPC: C12N9/-;C12N15/-; A61K38/-;A61P7/-

Documentation searched other than minimum documentation to the extent that such documents are included in the fields searched

Electronic data base consulted during the international search (name of data base and, where practicable, search terms used)

WPI, EPODOC, CNKI, CNPAT, SpringerLink, ISI Web of Knowledge, Elsevier Science Direct, Wiley InterScience, Pubmed, EMBL-EBI databases, GenBank, Tabanus yao, horsefly, protease?, fibrinogenolytic, tabfiblysin, enzyme, salivary gland?, platelet, aggregation, thrombosis, Dongying Ma, Ren Lai

**C. DOCUMENTS CONSIDERED TO BE RELEVANT**

| Category* | Citation of document, with indication, where appropriate, of the relevant passages | Relevant to claim No. |
| --- | --- | --- |
| PX | CN101608176A(KUNMING INSTITUTE OF ZOOLOGY CHINESE ACADEMY OF SCIENCES) 23 Dec.2009(23.12.2009) claims1-3 | 1-3 |
| A | XU, Xueqing et al., Toward an understanding of the molecular mechanism for successful blood feeding by coupling proteomics analysis with pharmacological testing of horsefly salivary glands, Molecular & Cellular Proteomics, 2008, Vol.7, No.3, pages 582-590 | 1-3 |
| A | ZHAO, Ruili et al., Immunoregulatory peptides from salivary glands of the horsefly, Tabanus pleskei, Comparative Biochemistry and Physiology, Part B, 28 March 2009, Vol. 154, pages 1-5 | 1-3 |

☒ Further documents are listed in the continuation of Box C.   ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim (S) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 24 Aug. 2010(24.08.2010) | **23 Sep. 2010 (23.09.2010)** |

| Name and mailing address of the ISA/CN | Authorized officer |
| --- | --- |
| The State Intellectual Property Office, the P.R.China 6 Xitucheng Rd., Jimen Bridge, Haidian District, Beijing, China 100088 | MA, Qiujuan |
| Facsimile No. 86-10-62019451 | Telephone No. (86-10) 82245903 |

Form PCT/ISA /210 (second sheet) (July 2009)

## INTERNATIONAL SEARCH REPORT

| | International application No. |
|---|---|
| | PCT/CN2010/073987 |

C (Continuation).     DOCUMENTS CONSIDERED TO BE RELEVANT

| Category* | Citation of document, with indication, where appropriate, of the relevant passages | Relevant to claim No. |
|---|---|---|
| A | KAZIMIROYA, M et al. Identification of anticoagulant activities in salivary gland extracts of four horsefly species (Diptera, Tabanidae), Haemostasis, 2001, Vol. 31, pages 294-305. | 1-3 |
| A | FRANCISCHETTI, Ivo M. B. et al., Purification, cloning, expression, and mechanism of action of a novel platelet aggregation inhibitor from the salivary gland of the blood-sucking bug, Rhodnius prolixus, The Journal of Biological Chemistry, 28 April 2000, Vol. 275, No. 17, pages 12639-12650 | 1-3 |
| A | YAN, Xiuwen et al., An immunoregulatory peptide from salivary glands of the horsefly, Hybomitra atriperoides, Developmental and Comparative Immunology, 2008, Vol. 32, pages 1242-1247 | 1-3 |
| A | XU, Xueqing et al., Antihemostatic molecules from saliva of blood-feeding Arthropods. Progress in Biochemistry and Biophysics, 2008, Vol. 35, No. 7, pages 757-765 | 1-3 |
| A | CN101220091A(KUNMING INSTITUTE OF ZOOLOGY CHINESE ACADEMY OF SCIENCES) 16 July 2008(16.07.2008) the whole document | 1-3 |
| A | WO00/11172A1(AUBURN UNIVERSITY) 02 March 2000(02.03.2000) the whole document | 1-3 |

Form PCT/ISA /210 (continuation of second sheet ) (July 2009)

**INTERNATIONAL SEARCH REPORT**
Information on patent family members

| | International application No. |
|---|---|
| | PCT/CN2010/073987 |

| Patent Documents referred in the Report | Publication Date | Patent Family | Publication Date |
|---|---|---|---|
| CN101608176A | 23.12.2009 | None | |
| CN101220091A | 16.07.2008 | None | |
| WO0011172A1 | 02.03.2000 | AU5778999A | 14.03.2000 |
| | | EP1105484A1 | 13.06.2001 |
| | | BR9913132A | 16.10.2001 |
| | | CN1320165A | 31.10.2001 |
| | | ZA200101375A | 24.04.2002 |
| | | US6451992B1 | 17.09.2002 |
| | | AU760288B | 08.05.2003 |
| | | US2003114654A1 | 19.06.2003 |
| | | MXPA01001849A | 01.05.2002 |
| | | US6927279B2 | 09.08.2005 |
| | | MX227166B | 08.04.2005 |
| | | CN1167796C | 22.09.2004 |
| | | EP1105484B1 | 03.05.2006 |
| | | DE69931167E | 08.06.2006 |
| | | ES2263286T3 | 01.12.2006 |
| | | DE69931167T2 | 22.02.2007 |

Form PCT/ISA /210 (patent family annex) (July 2009)

# INTERNATIONAL SEARCH REPORT

| International application No. |
| --- |
| PCT/CN2010/073987 |

Continuation of: A. CLASSIFICATION OF SUBJECT MATTER:

C12N9/64 (2006.01)i

C12N15/57 (2006.01)i

A61K38/48 (2006.01)i

A61P7/02 (2006.01)i

Form PCT/ISA /210 (extra sheet) (July 2009)